# EUROPÄISCHE PATENTSCHRIFT

(11) **EP 2 601 993 B1**
(45) Veröffentlichungstag und Bekanntmachung des Hinweises auf die Patenterteilung: **14.02.2018**
(21) Anmeldenummer: 12008115.3
(22) Anmeldetag: 05.12.2012
(51) Int. Cl.: A61M 16/06

(54) **Vorrichtung zur Positionierung eines Patienteninterface**
Device for positioning a patient interface
Dispositif destiné au positionnement d'une interface patient

(30) Priorität: 07.12.2011 DE 102011120367
(43) Veröffentlichungstag der Anmeldung: 12.06.2013
(73) Patentinhaber: Löwenstein Medical Technology S.A., 2557 Luxembourg (LU)
(72) Erfinder: Hahn, Henry, 25225 Hamburg (DE); Feldhahn, Karl-Andreas, 22761 Hamburg (DE); Gardein, Joachim, E-38430 Icod de los Vinos, Tenerife (ES)
(74) Vertreter: Marx, Thomas

(56) Entgegenhaltungen:
- EP-A2- 1 205 205
- EP-A2- 2 005 985
- WO-A1-2007/143793
- DE-A1-102010 021 276
- US-A1- 2004 112 384

## Beschreibung

### Gebiet der Erfindung

Die Erfindung betrifft eine Vorrichtung zur Positionierung eines Patienten Interfaces am Kopf des Anwenders.

### Stand der Technik

Patienten Interfaces dienen dazu, vom Beatmungsgerät, bereitgestelltes Atemgas an den Patienten abzugeben. Patienteninterfaces können in unterschiedlichen Ausführungsformen beispielsweise Nasal- oder Volfgesichtsmasken realisiert sein. Das Patienten Interface ist typischerweise über einen Atemgasschlauch mit dem Beatmungsgerät verbunden und wird am Kopf des Anwenders fixiert.

Das Patienten Interface muss stunden-, bzw. nächtelang auf dem Gesicht eines Patienten verweilen, wodurch hohe Anforderungen an den Tragekomfort gestellt sind.

Eine genaue Passform des Patienten Interfaces sowie der Befestigung ist erforderlich um eine Therapie-Beemträchtigung durch z.B. Verrutschen oder Lösen zu vermeiden. Ein schlecht sitzendes Patientenlnterface kann zu unangenehmen Druckstellen am Kopf des Patienten bis hin zu Undichtigkeiten bzw. Leckagen führen.

### Kritik am Stand der Technik

Zur Gewährleistung einer sicheren Positionierung der Atemmaske im Bereich des Gesichtes eines Patienten, sowie der Verringerung der Kräfte die auf das Gesicht wirken, werden Atemmasken mit Stirnabstützungen benutzt. Derartige Stirnstützen besitzen typischerweise Höhen- oder Abstandsverstellmöglichkeiten. Viele Verstelleinrichtungen sind für den Patienten aber oft zu kompliziert und sind meist nur aufwendig vom Patienten oder im Schlaflabor einstellbar.

Die WO 2007/143793 A1 offenbart eine Verstellvorrichtung für ein Patienteninterface, bei der das Betätigungselement ein Drehknopf ist. Das Außen-Gewinde des Drehknopfes greift in das komplementäre Teilgewinde des Verbindungselementes der Stirnabstützung. Der Drehknopf lässt sich in Raststufen bewegen. Eine Drehbewegung des Drehknopfes verschiebt die Stimabstützung gerastet vor oder zurück. Die EP 1 205 205 und US 2004/112384 offenbaren Verstellvorrichtungen für ein Patienteninterface bestehend aus einem Stützkörper mit einem Verbindungselement wobei das Verbindungselement in einer Aufnahme des Patienteninterface beweglich ist und dort über ein Betätigungselement mittels Rastung fixierbar ist wobei das Lösen der Rastung über ein Niederdrücken der Bedienflächen des Betätigungselementes erfolgt, welches dazu Griffflächen aufweist. Eine einfache, direkte und nicht gerastete Anpassung - möglichst einhändig durchführbar - ist für den Patienten selbst meist nicht möglich.

### Erfindung

Diese Erfindung betrifft eine Atemmaske, die eine Stirnabstützung aufweist Durch die zusätzliche Verwendung einer Stirnstütze an dem gaszuführenden Patienten Interface, wie z.B. einer Nasalmaske, wird die sichere Positionierung der Atemmaske im Bereich des Gesichtes eines Patienten gewährleistet. Ein ungewolltes Ablösen oder Verrutschen der Maske vom Gesicht des Benutzers und daraus folgende Leckagen werden vermieden. Das Verrutschen eines schlecht sitzenden Patienten Interfaces kann zu unangenehmen Druckstellen bis hin zu Therapiebeeinträchtigungen bzw. Therapieunterbrechungen führen. Sowohl der Tragekomfort als auch die Stabilität werden durch das Verwenden einer Stirnabstützung erhöht. Es lassen sich Druckstellen im Anlagebereich der Maske vermeiden, die bei einer Beatmungsmaske ohne Stirnabstützung durch die Vorspannung der Bänderung entstehen würden. Um den Druck auf den Nasenrücken zu verringern, ist jedoch eine individuelle Einstellung der Stirnstütze zur Maske erforderlich.

Aufgabe der vorliegenden Erfindung ist es daher, eine Vorrichtung zur Beatmung der einleitenden Art derart zu konstruieren, dass eine funktionelle, einfach zu bedienende Verstellung der Stirnabstützung bereitgestellt und dabei eine hohe Funktionalität und Robustheit gegeben wird, die den Tragekomfort eines Patienten Interfaces und die Compliance durch den Patienten zu verbessern hilft. Insbesondere muss die Position der Stirnstütze fein einstellbar sein und die eingestellte Position sicher gehalten werden.

Realisiert wird die Aufgabe durch eine Verstellvorrichtung für ein Patienteninterface bestehend aus einem Stützkörper mit einem Verbindungselement wobei das Verbindungselement in einer Aufnahme des Patienteninterface beweglich ist und dort über ein Betätigungselement mittels Klemmung fixierbar ist wobei das Betätigungselement als Klemmhülse ausgeführt ist und das Lösen der Klemmung über eine Drehbewegung der Klemmhülse erfolgt, welche dazu Griffflächen aufweist.

Die folgenden Figuren zeigen Ausführungsbeispiele eines Patienten Interfaces mit einer Stirnabstützung der einleitenden Art.
- Fig. 1 a - b:: Seitenansichten eines Patienteninterface mit verschiedenen Ausführungsformen des Stirnstützenschaftes
- Fig. 2:: Vorderansicht eines Patienten Interfaces mit Stirnabstützung
- Fig. 3 a - b:: perspektivische Explosionszeichnungen eines Patienten Interfaces mit verstellbarer Stirnstütze
- Fig. 4:: Perspektivansicht der Klemmhülse einer verstellbaren Stirnstütze
- Fig. 5:: perspektivische Explosionszeichnung eines weiteren Patierteninterfasces mit verstellbarer Stirnstütze
- Fig. 6:: Perspektivansicht der Flammhülse aus Fig. 5
- Fig. 7:: Perspektivansicht der Stirnabstützung aus Fig. 5

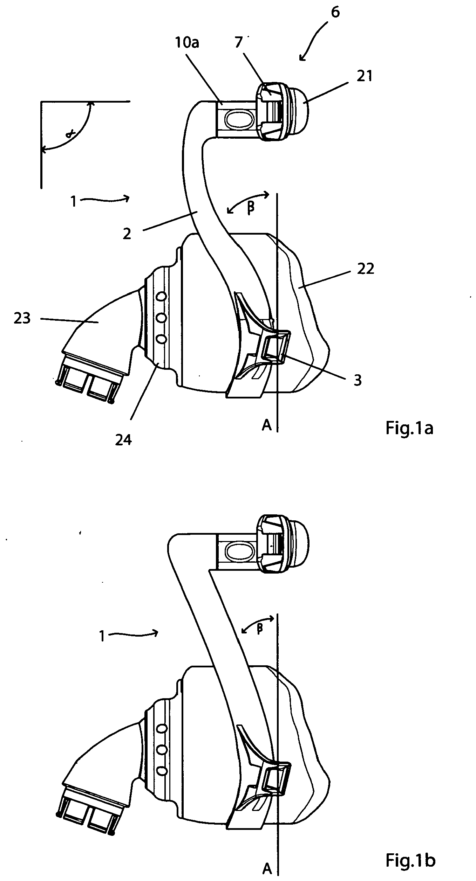

Fig.1 zeigt ein als Nasalmaske ausgebildetes Patienteninterface (1). Im Bereich ihrer dem Atemgasschlauch zugewandten Ausdehnung weist das Patienteninterface PI (1) eine Schlauchkupplung (23) auf. Eine Fixierung im Bereich eines Kopfes eines Patienten kann über eine Kopfhaube erfolgen. Die Bänderungsenden der Kopfhaube werden über die, auf beiden Seiten des PI vorgesehenen, Aufnahmevorrichtungen (3) für die Bänderung lösbar mit dem PI befestigt.
Die Schlauchkupplung (23) für den nicht dargestellten Atemgasschlauch ist mit einem Maskengrundkörper über ein Gelenk (24) verbunden, das kugelgelenkartig ausgebildet ist. Beim dargestellten Ausführungsbeispiel besteht das Gelenk (24) aus einem mit der Schlauchkupplung (23) verbundenen Innenteil sowie einer mit dem Maskengrundkörper verbundenen Außenschale. Das Innenteil ist wenigstens bereichsweise kugelsegmentartig ausgebildet und die Außenschale erstreckt sich entlang wenigstens eines Teiles der kugelsegmentartigen Oberfläche des Innenteiles. Am PI ist zur Abdichtung relativ zu einem Gesicht eines Patienten ein Dichtelement (22) angeordnet, das mindestens bereichsweise als Lippendichtung ausgebildet ist.

Der Stützkörper (6) ist hier als Stimabstützung (6) ausgeführt und besteht aus der Stirnpolsterauflage (21), die vom Stirnauflageträger (7) gehaltert wird. Ein Verstellelement (10a) verbindet die Stirnabstützung (6) lösbar und einstellbar mit dem Stirnstützenschaft (2). Der Stimstützenschaft (2) verbindet die Stirnabstützung (6) mit dem Grundkörper.

Der Stirnstützenschaft (2) weist in der Seitenansicht eine leicht gebogene Form (z.B. S-förmig) oder eine gerade als eine "7" ausgebildete Form auf, die im oberen Bereich vom Patientengesicht wegführt. Der Stimstützenschaft (2) ist gegenüber der senkrechten Ebene A um einen Winkel β von 1° bis 45°, bevorzugt um einen Winkel von 2° bis 30° vom Patientengesicht weg geneigt.

Im oberen Bereich des Stimstützenschaftes (2) befindet sich die Aufnahme (4), welche bevorzugt horizontal ausgeführt ist, für die Stirnabstützung (6). Die Aufnahme (4) steht im rechten Winkel zur Ebene A. Sie kann alternativ auch unter einem Winkel α, der zwischen 70° und 120° liegt, bevorzugt bei 80° bis 110° liegen kann, relativ zur Ebene A angeordnet sein. Es ist auch daran gedacht die Aufnahme gegenüber dem Stimstützenschaft (2) um einen Winkel α, der zwischen 70° und 120° liegt, schwenkbar zu gestalten.

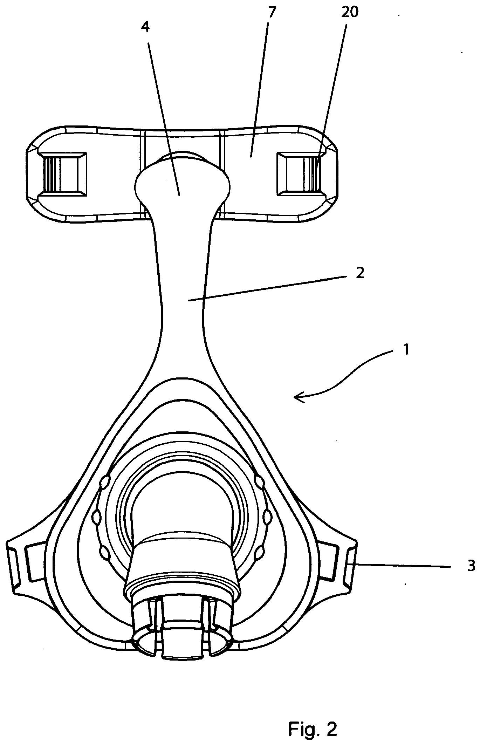

Fig.2 zeigt eine Vorderansicht des Patienteninterface. Hier ist erkennbar, dass der Stimstützenschaft (2) bereichsweise schmal ausgeführt ist, damit das Gesichtsfeld des Patienten nicht beeinträchtigt wird. Der schmalste Bereich des Stimstützenschaftes befindet sich, wenn das Patienteninterface an den Patienten angelegt ist, im Wesentlichen auf Augenhöhe. Der Stimstützenschaft (2) verjüngt sich ausgehend von Maskenkörper und verbreitert sich wieder zum oberen Bereich des Stimstützenschaftes (2) hin. Der obere Bereich des Stimstützenschaftes läuft in einer ellipsenförmigen Kontur (4) aus. Die Kontur (4) dient als Aufnahme für den Trägerarm (8) der Stirnstütze (6). Im Seitenbereich des Stirnauflageträgers (7) befinden sich Aufnahmen (20) für eine Bänderung.

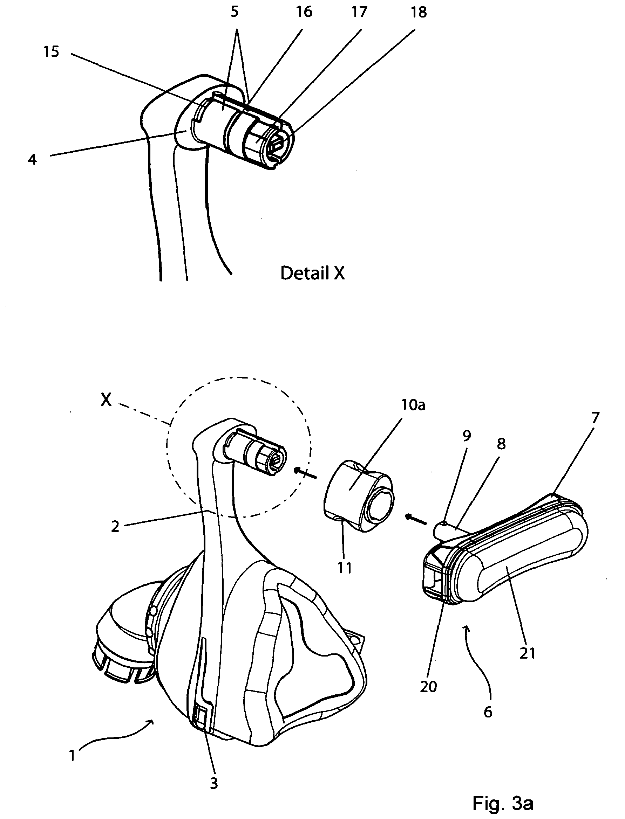

Fig.3a zeigt, dass die Stirnabstützung (6) über einem Arm (8), in eine Aufnahme (5) des Stimstützenschaftes (2) eingreift und dass eine Klemmhülse (10a) die Verbindung sichert.

Eine Verstellung der Stirnstütze (6) wird durch die zylindrische Aufnahme (5), über die eine Klemmhülse (10a) geschoben wird und durch den Trägerarm (8) der Stirnstütze (6), der in der Aufnahme (5) vor und zurück bewegt werden kann, erzielt.

Die Aufnahme besteht aus einem hohlen, zylindrischen, zweiteilig geschlitzten Außenteil (5), welches den Trägerarm (8) der Stirnstütze (6) aufnimmt. Im Inneren ist eine Führung (18) für den ebenfalls hohlen Trägerarm(19) angeordnet.

Die zylindrische Aufnahme (5) weist an der Außenseite einen Verrastungsanschlag (15) für die Klemmhülse (10a) auf, eine umlaufende Nut (16), die der Führung der Klemmhülse dient und die im Endbereich (17) einen kleineren Durchmesser aufweist, der in Richtung der Drehbewegung der Klemmhülse (10a) spiralförmig größer wird.

Die Klemmhülse (10a), die die gleiche elliptische Außenform aufweist wie der obere Teil des Stimstützenarmes (4) dient zum Klemmen des Trägerarms (8) in einer eingestellten Position.

Die Klemmhülse (10a) weist an den Außenseiten Griffmulden (11) auf, die ein einfaches Lösen der Hülse durch Drehung um ca. 45° - 60° ermöglichen. Im gelösten Zustand lässt sich der Trägerarm (8) leicht verschieben und durch Drehen der Hülse (10a) in die Ausgangsposition wird der Trägerarm (8) in der gewünschten Position fixiert. Die Position ist frei wählbar und nicht gerastet.

Zur Montage wird zuerst die Klemmhülse (10a) in der nicht klemmenden, entspannten Lage, die ca. 45° - 60° zur elliptischen Kontur des Stimstützenschaftes (4) steht, über das zylindrische Außenteil (5) geschoben, der Trägerarm (8) wird eingesetzt und in der gewählten Position durch Festdrehen der Klemmhülse (10a) gehalten.

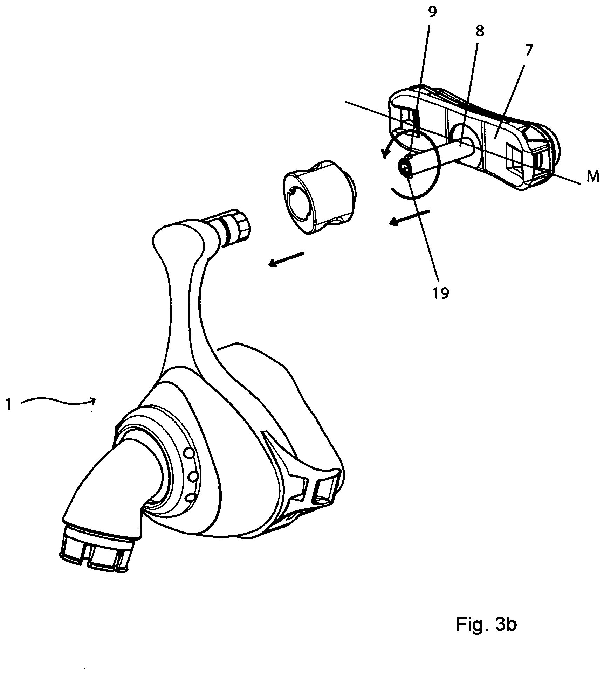

In Fig.3b ist erkennbar, dass der Trägerarm (8) an der Rückseite des Stimauflageträgers (7) horizontal außerhalb der Mitte (M) angebracht ist. Durch ein um 180° verdrehtes Einsetzen der Stirnstütze in die Aufnahme ist somit eine Höhenveränderung möglich.

Am Ende des Trägerarmes (8) sind Nasen oder Zähne (9) angebracht, die als Führung in den Schlitzen des zylindrischen Aufnahmeteiles (5) dienen und die bei der Verstellung einen Endanschlag bilden und verhindern, dass der Trägerarm (8) im gelösten Zustand ungewollt aus der Aufnahme (5) rutschen kann.

Die Oberfläche des Trägerarmes (8) kann eine elastomere Schicht z.B. eine Silikonbeschichtung aufweisen, die einen erhöhten Reibkoeffizienten bietet und dazu dient, ein ungewolltes Verrutschen des Trägerarmes zu verhindern. Gleiches kann durch eine angeraute Oberfläche erreicht werden.

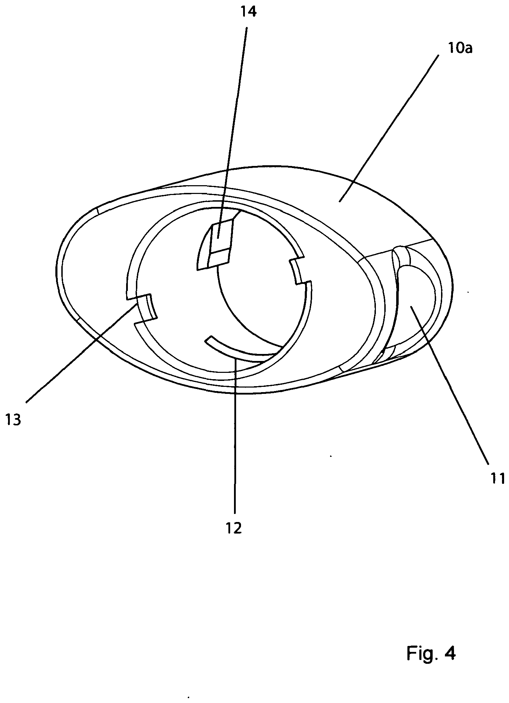

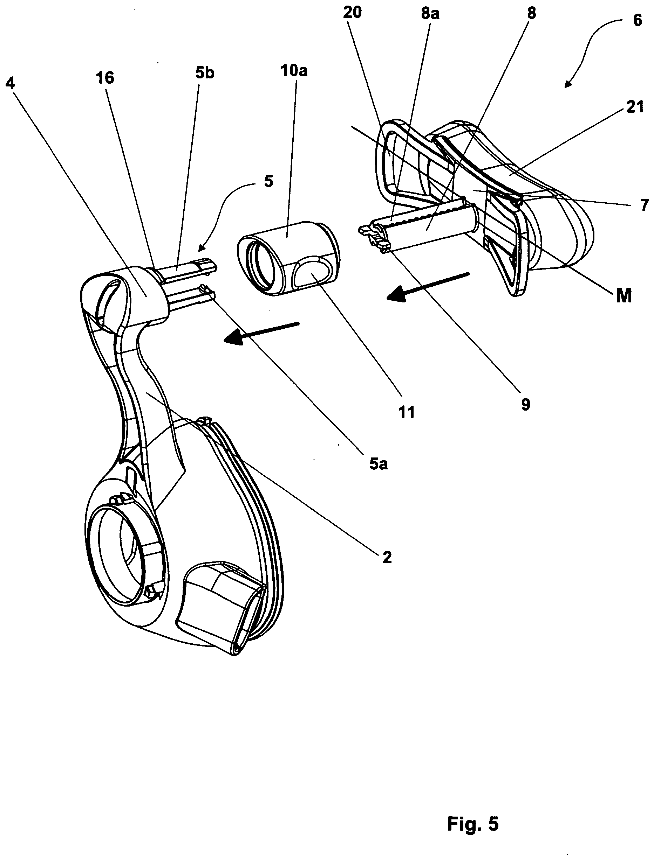

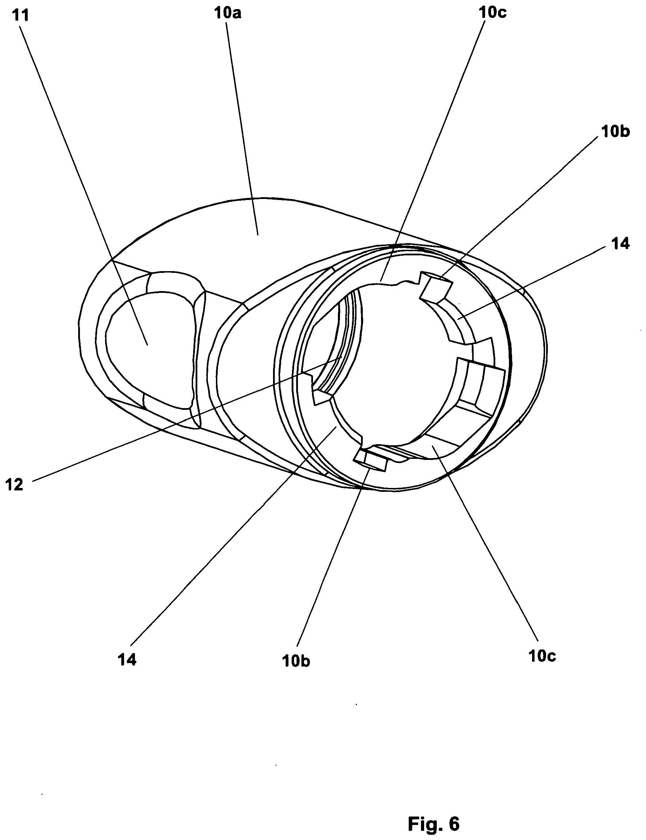

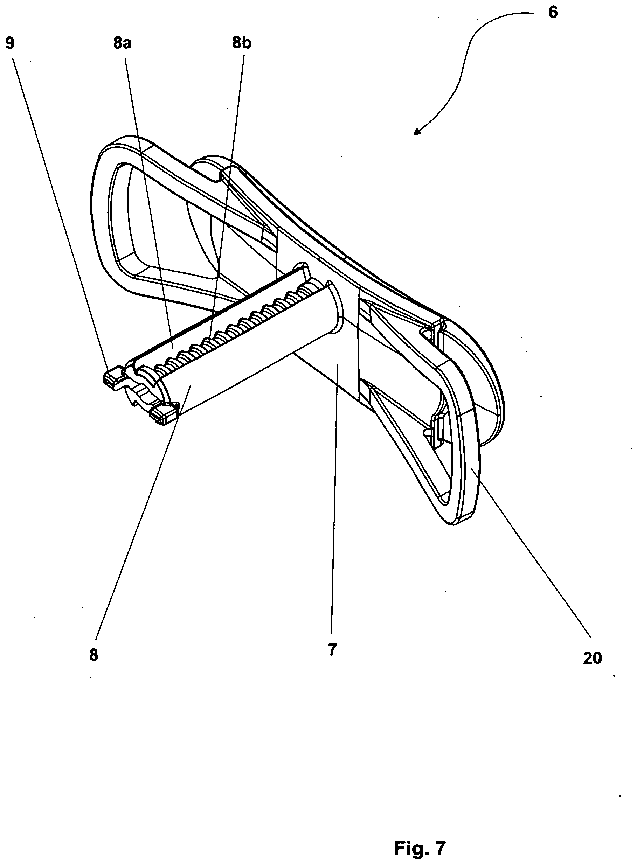

Fig.4 zeigt eine vergrößerte Ansicht der Klemmhülse (10a). Im Querschnitt ist die Hülse elliptisch und sie weist an den beiden Scheitelflächen Griffmulden (11) auf. Durch die exzentrische Anordnung der Griffmulden kann ein höheres Drehmoment bei einer Drehung der Hülse aufgebracht werden. Die Klemmhülse (10a) ist innen kreisrund, weist eine teilweise umlaufende Feder (12) auf, die in die umlaufende Nut (16) der zylindrischen Aufnahme (5) fasst und der Führung der Hülse (10a) dient. Sie hat an der dem Patienten abgewandten Seite vorspringende Elemente (13) z.B. als Nasen oder Zähne ausgebildet, angeordnet, die in den Verrastungsanschlag (15) der zylindrischen Aufnahme (5) fassen. Die vorspringende Elemente (13) dienen als Anschlag der Klemmhülse (10a) in der horizontalen Klemmlage. An der dem Patienten zugewandten Seite sind ebenfalls vorspringende Elemente (14) z.B. als Nasen oder Zähne ausgeführt, angebracht, die über das geschlitzte zylindrische Aufnahmeteil (5) ragen und beim Festdrehen der Klemmhülse (10a) die beiden federnden Hälften der zylindrischen Aufnahme (5) zusammendrücken und somit den eingefügten Trägerarm (8) der Stirnauflage (6) in seiner Position festklemmen. Der Drehweg der Klemmhülse (10a) beträgt ca. eine viertel bis eine halbe Umdrehung. Die Figuren 5-7 zeigen eine weitere Ausführungsform der erfindungsgemäßen Stirnstützenverstellung. Die Stirnabstützung (6) weist ebenfalls einen horizontal aus der Mitte (M) angebrachten Trägerarm (8) auf, um durch ein 180° verdrehtes Einsetzen der Stirnabstützung (6) eine Höhenverstellung zu ermöglichen.

Die Stirnabstützung (6) verfügt über einem Arm (8), der in eine Aufnahme (5) des Stirnstützenschaftes (2) eingreift. Die Verbindung wird über eine Klemmhülse (10a) gesichert.

Eine Verstellung der Stirnstütze (6) wird durch die Aufnahmestege (5b), über die eine Klemmhülse (10a) geschoben wird und durch den Trägerarm (8) der Stirnstütze (6), der in der Aufnahme (5) vor und zurück bewegt werden kann, erzielt.

Die Klemmhülse (10a) weist an den Außenseiten Griffmulden (11) auf, die ein einfaches Lösen der Hülse durch Drehung um ca. 45° - 60° ermöglichen. Im gelösten Zustand lässt sich der Trägerarm (8) leicht verschieben und durch Drehen der Hülse (10a) in die Ausgangsposition wird der Trägerarm (8) in der gewünschten Position fixiert. Die Position kann frei wählbar oder gerastet über Zähne (8b) in den Führungsnuten (8a) des Trägerarms (8) erfolgen.
Die Aufnahmestege (5b) weisen an der Außenseite einen ringförmigen Rand (16) auf. Dieser dient zur Positionierung der Klemmhülse (10a) auf der Aufnahme (5). Die Klemmhülse (10a) hat innen eine umlaufende Nut (12), die bei der Montage über den ringförmigen Rand (16) der Aufnahmestege (5b) greift. Dieses erfolgt indem man die Klemmhülse (10a) auf die Aufnahmestege (5b) bis zur ellipsenförmigen Kontur (4) drückt. Die Klemmhülse (10a) ist jetzt noch drehbeweglich kann aber axial nicht verrutschen oder verlorengehen.

Zur Montage der Stirnabstützung (6) muss die Klemmhülse (10a) in der nicht klemmenden, entspannten Lage, die ca. 45° - 60° zur elliptischen Kontur des Stimstützenschaftes (4) steht, gedreht sein, dann kann der Trägerarm (8) eingesetzt werden. Die Führungsnasen (9) des Trägerarms (8) müssen dabei durch die Aussparungen (10b) der Klemmhülse (10a) geführt werden und der Trägerarm (8) bis zur gewünschten Position in der Aufnahme (5) geschoben werden. Dann wird durch Drehen der Klemmhülse (10a) die Lage der Stirnabstützung (6) fixiert.

Der Trägerarm (8) weist oben und unten Führungsnuten (8a) auf. Sie dienen zur Aufnahme und Führung der Nasen (5a) der Aufnahmestege (5b) bei der Verstellung der Stimstütze, damit es nicht zu einem Verdrehen der Stirnabstützung kommt.

Die Klemmwirkung, um den Trägerarm (8) in der gewünschten Position zu fixieren, wird über die Schrägen (10c) in der Klemmhülse (10a) erzeugt, die beim Drehen in die "Festposition" der Klemmhülse (10a) die federnden Arme (5b) der Aufnahme (5) zusammendrückt. Dadurch wird die Nase (5a) der Aufnahme gegen die Zähne (8b) des Trägerarmes(8) gepresst. Die zusätzlichen Nasen (14) in der Klemmhülse (10a) bilden einen Endanschlag um den Trägerarm (8). Dieser kann dadurch nicht ungewollt aus der Aufnahme (5) und der Klemmhülse (10a) in der "Loseposition" gelöst werden.

Damit dem Patienten ein angenehmer Tragekomfort ermöglicht wird, bietet die Vorrichtung zur Beatmung eine Stimabstützung, dessen Stirnauflage eine Füllung aus einem weichen, flexiblen Material, z.B. ein Silikongel enthalten kann, die den Druck der Maske, bzw. der Bänderung abfängt. Der Maskenwulst kann ebenfalls eine Füllung aus einem weichen, flexiblen Material, z.B. ein Silikongel enthalten.

## Patentansprüche

1. Patienteninterface (1) mit einer Verstellvorrichtung bestehend aus einem Stützkörper (6) mit einem Verbindungselement (8) wobei der Stützkörper (6) als Stirnabstützung (6) ausgeführt ist und das Verbindungselement (8) als Trägerarm (8) ausgeführt ist und wobei das verbindungselement (8) in einer Aufnahme (5) des Patienteninterfaces (1) beweglich ist und dort über ein Betätigungselement (10a) mittels Klemmung fixierbar ist **dadurch gekennzeichnet, dass** das Betätigungselement als Klemmhülse (10a) ausgeführt ist und däss das Lösen der Klemmung über eine Drehbewegung der Klemmhülse (10a) erfolgt, welche dazu Griffflächen (11) aufweiset.

2. Patienteninterface (1) nach Anspruch 1 **dadurch gekennzeichnet, dass** das Betätigungselement (10a) das Verbindungselement (8) und die Aufnahme (5) zumindest abschnittsweise umgibt.

3. Patienteninterface (1) nach Anspruch 1 oder 2 **dadurch gekennzeichnet, dass** das Verbindungselement (8) in die Aufnahme (5) zumindest teilweise , eintaucht.

4. Patienteninterface (1) nach einem der vorhergehenden Ansprüche **dadurch gekennzeichnet, dass** die Klemmhülse (10a) innen eine Aufnahme mit einer teilweise umlaufende Feder (12) aufweiset.

5. Patienteninterface (1) nach einem der vorhergehenden Ansprüche **dadurch gekennzeichnet, dass** die Aufnahme (5) zwei Federelemente (5b) aufweist.

6. Patienteninterface (1) nach Anspruch 4 und/oder 5 dadurch gekennzeichet, dass die Aufnahme (5) einen Verrastanschlag (15) für die Klemmhülse (10a) aufweist.

7. Patienteninterface (1) nach einem der Ansprüche 4 bis 6 dadurch gekenntzeichnet, dass die Aufnahme (5) außen eine umlaufende Nut und/oder einen Rand (16) zur Führung der Klemmhülse (10a) aufweist.

8. Patienteninterface (1) nach einem der Ansprüche 4 bis 7 **dadurch gekennzeichnet, dass** im Endbereich (17) der Aufnahme (5) ein kleiner Durchmesser, der spiralförmig in Drehbewegung der Klemmhülse (10a) grösser wird angeordnet ist und somit durch Drehen der Klemmhülse (10a) das Klemmen des Trägerarms (8) in der vorgegebenen Position bewirkt.

9. Patienteninterface (1) nach Anspruch 5 oder einem der Ansprüche 6 bis 8 wenn abhängig von Anspruch 5 **dadurch gekennzeichnet, dass** die Klemmwirkung der Aufnahme auf den Trägerarm (8) über gegenüberliegende Schrägen (10c) in der Klemmhülse (10a) erzeugt wird, die beim Drehen in die "Festposition" der Klemmhülse (10a) die Federelemente (5b) der Aufnahme (5) zusammendrückt.

10. Patienteninterface (1) nach einem der vorhergehenden Ansprüche **dadurch gekennzeichnet, dass** zusätzliche Nasen (14) in der Klemmhülse (10a) einen Endanschlag bilden, um den Trägerarm (8) der Stirnabstützung (6) nicht ungewollt aus der Aufnahme (5) und der Klemmhülse (10a) in der "Loseposition" ziehen zu können.

11. Patienteninterface (1) nach einem der vorhergehenden Ansprüche **dadurch gekennzeichnet, dass** der Trägerarm (8) zumindest eine Führungsnut (8a) für ein Gegenstück der Aufnahme (5) aufweist.

12. Patienteninterface (1) nach einem der vorhergehenden Ansprüche **dadurch gekennzeichnet, dass** im Bereich der Klemmstelle ein erhöhter Reibkoeffizienten vorliegt.

13. Patienteninterface (1) nach Anspruch 12 **dadurch gekennzeichnet, dass** der Reibkoeffizient durch eine elastomere Beschichtung erhöht ist.

14. Patienteninterface (1) nach einem der vorhergehenden Ansprüche **dadurch gekennzeichnet, dass** eine Höhenverstellung der stirnabstützung (6) durch den horizontal aus der Mitte (M) angeordneten Trägerarm (8), der wahlweise um 180 [deg.] verdreht eingesetzt werden kann, erzielt wird.

## Claims

1. A patient interface (1) with an adjustment device consisting of a supporting body (6) with a connection element (8), wherein the supporting body (6) is designed as a front support (6) and the connection element (8) is designed as a support arm (8) and wherein the connection element (8) is movable in a receiver (5) of the patient interface (1) and is immobilizable there via an actuation element (10a) by means of a clamp, **characterized in that** the actuation element is designed as a clamping sleeve (10a) and that the loosening of the clamp takes place via a rotational movement of the clamping sleeve (10a), which has grip surfaces (11) for this purpose.

2. The patient interface (1) according to claim 1, **characterized in that** the actuation element (10a) surrounds the connection element (8) and surrounds the receiver (5) at least in sections.

3. The patient interface (1) according to claim 1 or 2, **characterized in that** the connection element (8) immerses at least partially into the receiver (5).

4. The patient interface (1) according to one of the preceding claims, **characterized in that** the clamping sleeve (10a) on the inside has a receiver with a partially circumferential spring (12).

5. The patient interface (1) according to one of the preceding claims, **characterized in that** the receiver (5) has two spring elements (5b).

6. The patient interface (1) according to claim 4 and/or 5, **characterized in that** the receiver (5) has a locking stop (15) for the clamping sleeve (10a).

7. The patient interface (1) according to one of claims 4 to 6, **characterized in that** the receiver (5) on the outside has a circumferential groove and/or a border (16) for guiding the clamping sleeve (10a).

8. A device according to one of claims 4 to 7, **characterized in that** a small diameter, which increases in a spiral-shaped manner in the rotational movement of the clamping sleeve (10a), is arranged in the end area (17) of the receiver (5) and the clamping of the support arm (8) is effectuated in the specified position by the rotation of the clamping sleeve (10a).

9. The patient interface (1) according to claim 5 or one of claims 6 to 8 if dependent on claim 5, **characterized in that** the clamping effect of the receiver (5) is generated on the support arm (8) via opposite-lying bevels (10c) in the clamping sleeve (10a), which pushes together the spring elements (5b) of the receiver (5) during the process of rotating into the "fixed position" of the clamping sleeve (10a).

10. The patient interface (1) according to one of the preceding claims, **characterized in that** additional noses (14) in the clamping sleeve (10a) form an end stop in order to prevent inadvertently pulling the support arm (8) of the front support (6) out of the receiver (5) and the clamping sleeve (10a) in the "loose position."

11. The patient interface (1) according to one of the preceding claims, **characterized in that** the support arm (8) has at least one guiding groove (8a) for a counterpiece of the receiver (5).

12. The patient interface (1) according to one of the preceding claims, **characterized in that** an increased friction coefficient is present in the area of the clamping unit.

13. The patient interface (1) according to claim 12, **characterized in that** the friction coefficient is increased by an elastomer coating.

14. The patient interface (1) according to one of the preceding claims, **characterized in that** a height adjustment of the front support (6) is achieved by the support arm (8) arranged horizontally from the middle (M), which can optionally be inserted rotated by 180[deg.].

## Revendications

1. Interface de patient (1) comprenant un dispositif de réglage constitué d'un corps de support (6) avec un élément d'assemblage (8), le corps de support (6) étant conçu comme un support de front (6) et l'élément d'assemblage (8) étant conçu comme un bras porteur (8), et dans lequel l'élément d'assemblage (8) est déplaçable dans un logement (5) de l'interface de patient (1), où il peut être fixé par serrage à l'aide d'un élément d'actionnement (10a), **caractérisée en ce que** l'élément d'actionnement est conçu comme une douille de serrage (10a) et **en ce que** le relâchement du serrage est effectué par un mouvement de rotation de la douille de serrage (10a), celle-ci présentant des surfaces de préhension (11) à cette fin.

2. Interface de patient (1) selon la revendication 1, **caractérisée en ce que** l'élément d'actionnement (10a) entoure au moins partiellement l'élément d'assemblage (8) et le logement (5).

3. Interface de patient (1) selon la revendication 1 ou 2, **caractérisée en ce que** l'élément d'assemblage (8) plonge au moins partiellement dans le logement (5).

4. Interface de patient (1) selon l'une des revendications précédentes, **caractérisée en ce que** du côté intérieur, la douille de serrage (10a) présente un logement avec un ressort (12) partiellement périphérique.

5. Interface de patient (1) selon l'une des revendications précédentes, **caractérisée en ce que** le logement (5) présente deux éléments de ressort (5b).

6. Interface de patient (1) selon la revendication 4 et/ou 5, **caractérisée en ce que** le logement (5) présente une butée d'enclenchement (15) pour la douille de serrage (10a).

7. Interface de patient (1) selon l'une des revendications 4 à 6, **caractérisée en ce que** du côté extérieur, le logement (5) présente une rainure périphérique et/ou un bord (16) pour le guidage de la douille de serrage (10a).

8. Dispositif selon l'une des revendications 4 à 7, **caractérisé en ce que** dans la région d'extrémité (17) du logement (5), il est prévu un petit diamètre augmentant en forme de spirale avec le mouvement de rotation de la douille de serrage (10a), et assurant ainsi le serrage du bras porteur (8) dans la position prédéfinie par rotation de la douille de serrage (10a).

9. Interface de patient (1) selon la revendication 5 ou l'une des revendications 6 à 8 dans la mesure où celle-ci dépend de la revendication 5, **caractérisée en ce que** l'action de serrage du logement (5) sur le bras porteur (8) est réalisée par des chanfreins opposés (10c) dans la douille de serrage (10a), laquelle comprime les éléments de ressort (5b) du logement (5) lors de la rotation dans la « position fixe » de la douille de serrage (10a)

10. Interface de patient (1) selon l'une des revendications précédentes, **caractérisée en ce que** des nez supplémentaires (14) forment une butée finale dans la douille de serrage (10a), pour éviter le retrait involontaire du bras porteur (8) du support de front (6) hors du logement (5) et de la douille de serrage (10a) dans la « position détachée ».

11. Interface de patient (1) selon l'une des revendications précédentes, **caractérisée en ce que** le bras porteur (8) présente au moins une rainure de guidage (8a) pour une contre-pièce du logement (5).

12. Interface de patient (1) selon l'une des revendications précédentes, **caractérisée en ce que** dans la région du point de serrage, le coefficient de friction est augmenté.

13. Interface de patient (1) selon la revendication 12, **caractérisée en ce que** le coefficient de friction est augmenté par un revêtement élastomère.

14. Interface de patient (1) selon l'une des revendications précédentes, **caractérisée en ce qu'**un réglage en hauteur du support de front (6) est obtenu à l'aide du bras porteur (8) disposé horizontalement à partir du milieu (M), lequel peut facultativement être utilisé en étant tourné de 180 degrés.
